# EUROPEAN PATENT APPLICATION

(11) **EP 2 128 824 A1**
(43) Date of publication of application: **02.12.2009**
(21) Application number: 07737536.8
(22) Date of filing: 28.02.2007
(51) Int. Cl.: G06T 15/00

(54) **GRAPHICS PLOTTING DEVICE AND GRAPHICS PLOTTING METHOD**

(71) Applicant: Panasonic Corporation, Kadoma-shi Osaka 571-8501 (JP)
(72) Inventor: HARUMOTO. Hideaki, Chuo-ku Osaka 540-6207 (JP)
(74) Representative: Grünecker, Kinkeldey, Stockmair & Schwanhäusser Anwaltssozietät
(86) International application number: PCT/JP2007/053817
(87) International publication number: WO 2008/105092

(57) **Abstract**

When moving images such as three-dimensional graphics, or the like are displayed, high-quality images can be displayed by keeping a display frame rate, suppressing a breakdown of display contents, and making a drawing engine exhibit processing abilities to maximum. A graphics rendering device includes a frame buffer 40 in which a plurality of areas are provided, a rendering frame counter 15 for counting a number of frames in which the rendering is ended, a display frame counter 17 for counting a number of frames in which a display on a screen is completed within respective frames whose rendering is ended, a rendering controlling portion 11 for determining whether or not an empty area is present on the frame buffer based on the value of the rendering frame counter and the value of the display frame counter and permitting the rendering of the display element in the next frame when the empty area is present, a rendering necessary-time predicting portion 13 for predicting a rendering required time, and a rendering omission controlling portion 12 for determining whether or not the rendering is completed before display based on the value of the display frame counter, a display period of the frame, and a predicted rendering required time and going to a process in the next frame by omitting an execution of a succeeding rendering command when the rendering is not completed.

## Description

### Technical Field

The present invention relates to a graphics rendering device and a graphics rendering method for rendering sequentially display elements such as graphics, characters, images, etc., which are to be displayed in synchronism with a plurality of display frames that appear sequentially in time series respectively, in compliance with any input commands.

### Background Art

For example, most of the equipments such as the cellular phone terminal, the mobile information terminal (PDA), and the like can execute various applications in case of necessity. Out of the executable application programs, many programs that display various graphics or characters on a screen by utilizing three-dimensional graphics, e.g., the 3D game utilizing various characters displayed in three dimensions, the navigation displaying the three-dimensional map, the simulator displaying the three-dimensional graphics, and the like, are contained.

Also, the moving images must be displayed on a screen in the above application programs, and the contents displayed on the screen by the application program are updated frequently in a short time period. In particular, when the application programs such as the games, or the like intend to display the high-quality moving images that changes smoothly, the contents of the images that are to be displayed on the screen at a predetermined frame period must be updated at a high speed.

In this event, very high rendering processing capabilities are needed to render the image such as the three- dimensional graphics. Also, the time that is allowable for the rendering operation of each frame constituting the moving images is limited by the frame period, and therefore a huge amount of display elements must be rendered within a very short time.

In the computer graphics device, not only there is such a demand that fine images should be rendered at as high definition as possible and but also there is such a demand that the frame rate of display should be held as highly as possible. If it is acceptable to lower the display rate, high-definition beautiful images may be rendered slowly. However, when the images involving a lot of motions must be rendered in the game application, or the like, the appropriate presentation could not be provided to the user unless the frame rate can be held.

The method of adjusting the rendering performance of the computer graphics system, which is devised to satisfy such demand, is disclosed in Patent Literature 1, for example. In Patent Literature 1, the simulation algorithm used to calculate a time required for the rendering operation of the display elements is offered. This algorithm aims at creating such circumstances that the unreasonable rendering process that make it impossible to keep the frame rate should not be applied from the beginning, by causing the creator who creates the information acting as the source to execute the rendering process, i.e., the game developer of the game application, to choose optimum rendering parameters.

Also, in the prior art disclosed in Patent Literature 2, such an approach has been proposed that the number of produced polygons of the three-dimensional characters should be controlled based on the rendering capabilities of the rendering engine in response to a looking layout in an area occupied on the screen in such a manner that an amount of rendering operations assigned to one frame can be suppressed within the rendering capabilities of the rendering engine.
Patent Literature 1: JP-T-2002-503854
Patent Literature 2: JP-A-2000-242807

### Disclosure of the Invention

### Problems that the Invention is to Solve

For example, in the situation that the rendering engine whose processing capabilities can render 5000 display elements within a time corresponding to a frame period is employed, actually the display elements of total data cannot rendered within an allowable time when the data for the command that needs the display of 10000 display elements in one frame are prepared. Therefore, the contents in which a part of the displayed images is lost are displayed on the screen, and thus a breakdown of the display contents is caused. When the conventional method disclosed in Patent Literature 1 is employed, the creator can prepare in advance the complete data that do not cause a breakdown of the display, prior to the rendering process.

Also, the user can utilize such prior art to decide in real time whether or not the display rate can be held, at a time of executing the rendering process of the contents software when such user downloads the game contents, or the like by using the cellular phone terminal, or the like, for example. However, such prior art cannot provide the concrete countermeasure for the keeping of the display rate itself.

In particular, the models of the cellular phone terminal range widely, and it is highly possible that the rendering capabilities of the rendering engines that are installed into respective cellular phone terminals to render should be largely different every model. Therefore, even when the contents such as the games, or the like oriented to the particular model to keep the display rate are prepared, such a case may happen that the contents cannot be reproduced correctly because the rendering capabilities are inferior when the model is different.

Also, when the prior art in Patent Literature 2 is employed, the frame rate of the display can be held by executing the decimation of the polygon rendering. However, upon executing the decimation, the assignment of the number of polygons of the three-dimensional characters is decided only based on the capabilities of the used rendering engine. As a result, it is feared that the decimation that the contents creator can permit is not always realized. In other words, the number of rendered polygons is deleted more than necessary to keep the frame rate of the display, and thus there is such a possibility that the screen of the image quality that the creator of the contents desires cannot be displayed.

It is an object of the present invention to provide a graphics rendering device and a graphics rendering method, capable of displaying high-quality images by keeping a display frame rate, suppressing a breakdown of display contents, and making a drawing engine exhibit processing abilities to maximum even when an allowable time for a rendering operation in each frame is short in such a situation that moving images such as three-dimensional graphics, or the like, which demand high rendering processing capabilities, should be displayed.

### Means for Solving the Problems

A graphics rendering device of the present invention for sequentially rendering display elements such as graphics, characters, images, and the like, which are to be displayed in synchronism with a plurality of display frames which appear sequentially in time series respectively, in compliance with any input commands, includes a frame buffer having a plurality of areas for plural frames, each of the areas storing data of display contents displayed in one display frame; a rendering frame counter for counting a value which is changed in response to a number of frames in which the rendering is ended; a display frame counter for counting a value which is changed in response to a number of frames in which a display on a screen is completed within respective frames whose rendering is ended; a rendering controlling portion for determining whether or not an empty area is present on the frame buffer based on the value of the rendering frame counter and the value of the display frame counter, and permitting the rendering of the display element in the next frame when it is determined that the empty area is present; a rendering necessary-time predicting portion for predicting a rendering required time when the rendering controlling portion permits the rendering; and a rendering omission controlling portion for determining whether or not the rendering is completed before the display, based on the value of the display frame counter, a display period of the frame, and a rendering required time predicted by the rendering necessary-time predicting portion, and performing a rendering process of the next frame by omitting an execution of a succeeding rendering command when it is determined that the rendering is not completed before the display.

According to this configuration, the frame buffer has the areas that store the data of the display contents and correspond to the plural frames. Therefore, the rendering process is applied to respective areas of the frame buffer before the images in respective frames are displayed on the screen, and the contents of the frame buffer in respective areas whose rendering operation is completed can be displayed on the screen at necessary timings. As a result, while the image in one frame is displayed on the screen, the rendering operations of the image in a plurality of other frames can be executed respectively. For example, even when the image in each frame is displayed sequentially on the screen in a predetermined frame period, the contents rendered in one frame time are not restricted within one frame, and the images ranging over two frames or more can be rendered in one frame time if there is room for the actual rendering processing capabilities. In other words, if the rendering of the display contents in the frame that is to be displayed next at certain timing is finished, the rendering of the display contents to be displayed after next can be started. As a result, the rendering process can be executed effectively by reducing a waiting time in which the rendering operation is not actually executed, and thus the processing capabilities of the rendering engine can be exhibited to maximum.

In this case, the number of areas on the frame buffer is finite, and therefore the particular number of areas must be used in sequence repeatedly in a circulative manner respectively. For this reason, when the number of display elements displayed in each frame is relatively small rather than the actual rendering processing capabilities, it is feared that, after the rendering is completed, the overwriting of the rendering contents is applied to the area in the frame, in which the display is not ended yet, by the further rendering process.

In order to prevent such situation, the rendering frame counter, the display frame counter, and a rendering controlling portion are provided. The rendering frame counter counts the value which is changed in response to the number of frames in which the rendering is ended. The display frame counter counts the value which is changed in response to the number of frames in which the display on the screen is completed within respective frames whose rendering is ended. The rendering controlling portion determines whether or not an empty area (the area in which the data whose display has been finished is held is regarded as the empty area) is present on the frame buffer based on the value of the rendering frame counter and the value of the display frame counter, and then permits the rendering of the display element in the next frame when it is determined that the empty area is present.

Meanwhile, when the process of displaying the contents in respective areas on the screen is applied after various display elements to be displayed are rendered on respective areas on the frame buffer, the rendering process must be ended every display frame immediately before the contents in respective areas are displayed on the screen. However, the actual rendering processing capabilities are different every type of the employed rendering engine, and the required times required for the actual rendering process of the rendering commands and the data as the rendered object are not assumed previously. Therefore, all rendering commands as the rendered object cannot always be processed within a limited time (until the display of the concerned frame is started).

Therefore, the rendering omission controlling portion determines whether or not the rendering is completed before the display, based on the value of the display frame counter, the display period of the frame, and the rendering required time predicted by the rendering necessary-time predicting portion, and performing a rendering process of the next frame by omitting an execution of the succeeding rendering command when the rendering is not completed before the display. Accordingly, the maximum rendering process can be executed within a range in which the rendering can be completed up to the display timing of the concerned frame. As a result, even though a huge amount of rendering commands are prepared to display the high-quality images, the rendering process can be punctuated within an allowable time for each frame, and consequently a total quality of the displayed images can be maintained so-so.

Also, in the graphics rendering device of the present invention, the rendering necessary-time predicting portion estimates a required time for the rendering in one frame based on the list of the rendering commands indicating the rendering contents in the concerned frame, as a cumulative value of constants indicating execution required times that are assigned in advance to respective rendering commands contained in the list.

According to this configuration, a cumulative value of the required times detected when respective rendering commands are executed is calculated. Therefore, even when the rendering processing capabilities of the actually employed rendering engine are changed, a required time for the rendering in one frame can be estimated precisely.

Also, in the graphics rendering device of the present invention, the rendering necessary-time predicting portion estimates a required time for the rendering in one frame based on a list of the rendering commands indicating the rendering contents in the concerned frame, as a cumulative value of the required times detected when respective rendering commands contained in the list are executed.

The required times needed when a predetermined rendering engine should execute respective rendering commands as the processed object are changed largely depending on the processing capabilities of the rendering engine itself and the type of the rendering command. When the rendering engine whose processing capabilities have already been known is employed, a required time for every rendering command can be estimated by the calculation made based on the constants that can be specified every type of the rendering command.

According to this configuration, a required time for the rendering operation in one frame can be estimated by calculating a cumulative value of the constants that are specified every type of the rendering command.

Also, the graphics rendering device of the present invention further includes a drawing command list holding portion for holding the list of the rendering commands indicating the rendering contents in respective frames, and holding priority information indicating categories concerning two types or more of priorities in the list every rendering command; wherein the rendering controlling portion determines whether or not respective rendering commands contained in the list held in the drawing command list holding portion are able to execute the rendering, based on the correlated priority and processing capabilities of a rendering engine that executes the rendering, and omits an execution of the rendering commands that satisfy predetermined conditions.

When the process of displaying the contents in respective areas on the screen is applied after various display elements to be displayed are rendered on respective areas on the frame buffer, the rendering process must be ended every display frame immediately before the contents in respective areas are displayed on the screen. However, the actual rendering processing capabilities are different every type of the employed rendering engine, and also the required times for the actual rendering process of the rendering commands and the data as the rendered object are not assumed previously. Therefore, all rendering commands as the rendered objects cannot always be processed within a limited time (until the display of the concerned frame is started).

However, since it is desirable that the image of the higher quality should be displayed in so far as it is possible, the data of the display objects (the list of the rendering commands) must be prepared extensively with as high precision as possible. However, such a situation may occur that all rendering commands cannot be executed within an allowable time on account of the limits of the rendering processing capabilities of the rendering engine employed actually. In such case, the breakdown of the display contents is brought about, and thus there is such a possibility that the screen of quality that is desired by the contents creator (for example, the game creator) is not displayed.

According to this configuration, priority information can be provided to the rendering commands in the list every rendering command. Therefore, the rendering command having the high priority can be handled preferentially such that the rendering is not omitted to the extent possible. Also, it is determined whether or not the rendering operation can be executed, based on the priority and the processing capabilities of the rendering engine. Therefore, the device employing the rendering engine whose processing capabilities are set high can also handle the rendering commands with low priority in such a way that their rendering should not be omitted. As a result, the contents creator can reflect his or her own intension (the contents to be displayed on a preferential basis even if the performance of the rendering engine is low) onto the priority of each rendering command, and can suppress the breakdown of the display contents.

Also, a graphics rendering method of the present invention of rendering sequentially display elements such as graphics, characters, images, and the like, which are to be displayed in synchronism with a plurality of display frames that appear sequentially in time series respectively, in compliance with any input commands, includes utilizing a frame buffer in which an area for storing data of display contents displayed in one display frame is provided to correspond to plural frames; counting a first value that is changed in response to a number of frames in which the rendering is ended; counting a second value that is changed in response to a number of frames in which a display on a screen is completed within respective frames whose rendering is ended; determining whether or not an empty area is present on the frame buffer, based on the first value and the second value, and permitting the rendering process of the display element in a succeeding frame and predicting a rendering required time when the empty area is present; and determining whether or not the rendering is completed before the display, based on the second value, a display period of the frame, and a predicted rendering required time, and for going to a rendering process of a next frame by omitting an execution of a succeeding rendering command when the rendering is not completed before the display.

According to this method, such a control is applied that it is determined whether or not the rendering is completed before the display, and the process goes to the rendering process in the next frame by omitting the execution of the succeeding rendering command when it is predicted that the rendering is not completed before the display. Therefore, the maximum rendering process can be executed within a range in which the rendering can catch up with the display timing of the concerned frame. As a result, even though a large amount of rendering commands are prepared to display the high-quality images, the rendering process can be punctuated within an allowable time for each frame, and consequently a total quality of the displayed image can be maintained at a reasonable level.

### Advantages of the Invention

According to the present invention, such a control is applied that, even when even when an allowable time for a rendering operation in each frame is short in such a situation that the moving images such as three-dimensional graphics, or the like, which need high rendering processing capabilities, should be rendered, it is determined whether or not the rendering is completed before the display and then the process goes to the rendering process in the succeeding frame by omitting the execution of the succeeding rendering command if the rendering is not completed before the display. As a result, the display frame rate can be kept and also a breakdown of display contents can be suppressed, so that a total quality of the display image can be maintained so-so. Also, a high-quality image can be displayed by making the drawing engine exhibit the processing abilities to maximum.

### Brief Description of the Drawings

[FIG.1] A block diagram shows an example of a hardware configuration of a graphics display device according to an embodiment.
[FIG.2] Flowcharts show examples of operations of a principal portion of the device shown in FIG.1.
[FIG.3] A time chart shows an example of an operation of the device shown in FIG.1 and an operation in the prior art.
[FIG.4] A time chart shows an example of an operation of the device shown in FIG.1.
[FIG.5] A time chart shows an example of an operation of the device shown in FIG.1 and an operation in the prior art.
[FIG.6] A block diagram shows a concrete example of constituent elements used to detect a rendering necessary time.
[FIG.7] A schematic view shows a configurative view of a rendering command list.
[FIG.8] A flowchart shows an example of an operation of the principal portion of the device shown in FIG.1.

### Description of Reference Numerals

- 10: graphics processing portion
- 11: microprocessor
- 12: polygon rendering engine
- 13: rendering time detecting portion
- 14: rendering-time accumulation calculating portion
- 15: rendering counter
- 16: displaying engine
- 17: display counter
- 20: rendering command holding portion
- 21: rendering command list
- 30: display device
- 40: ring buffer
- 41: memory area

### Best Mode for Carrying Out the Invention

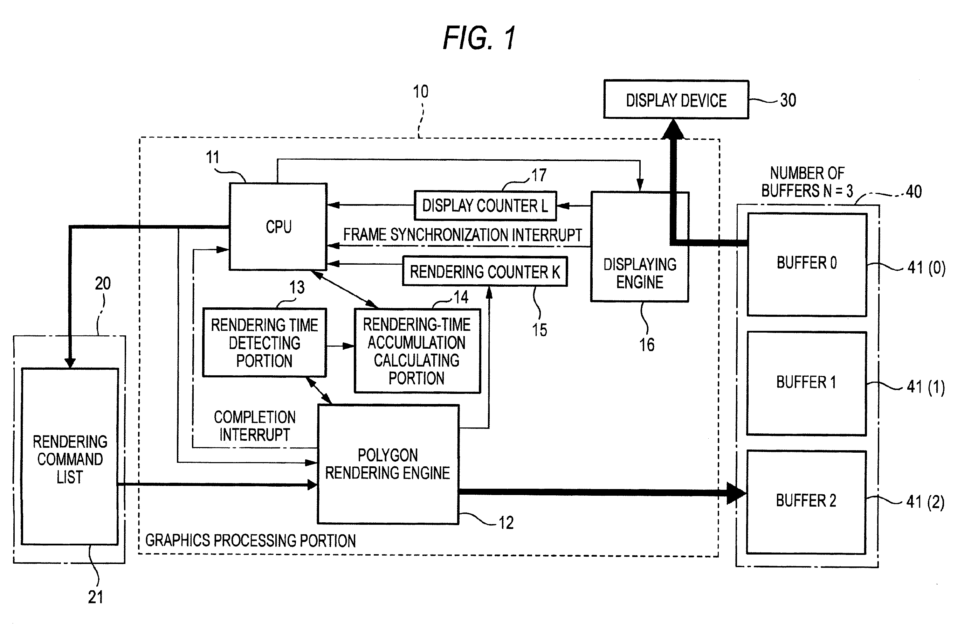

One concrete embodiment of a graphics rendering device and a graphics rendering method of the present invention will be explained with reference to FIG.1 to FIG.8 hereinafter.

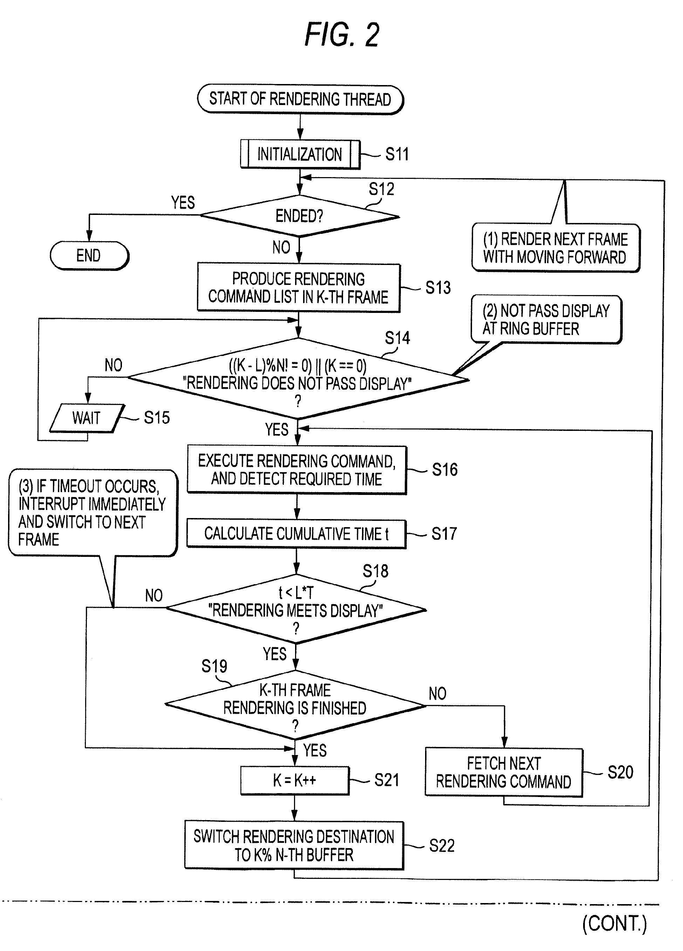

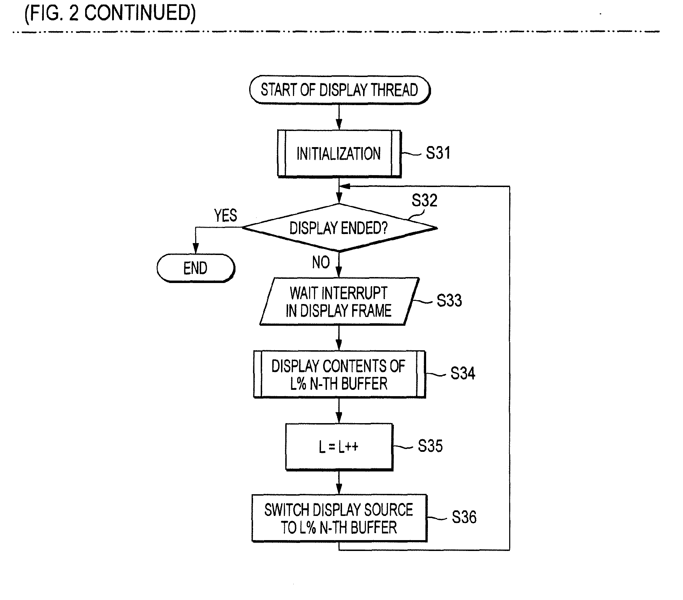

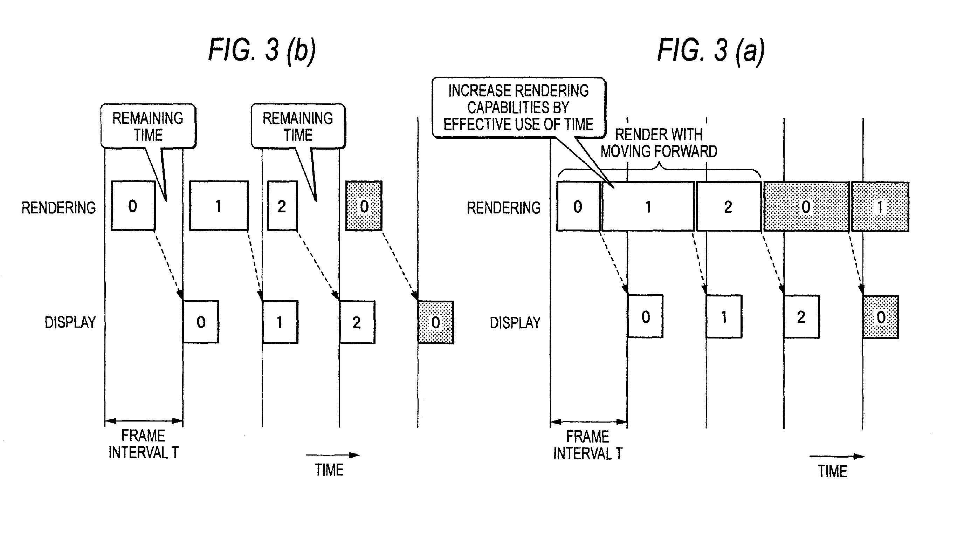

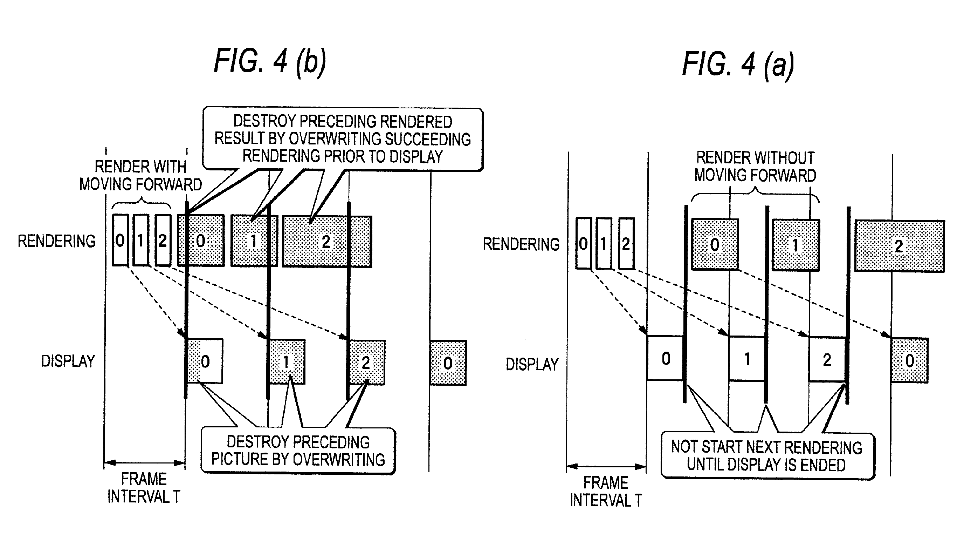

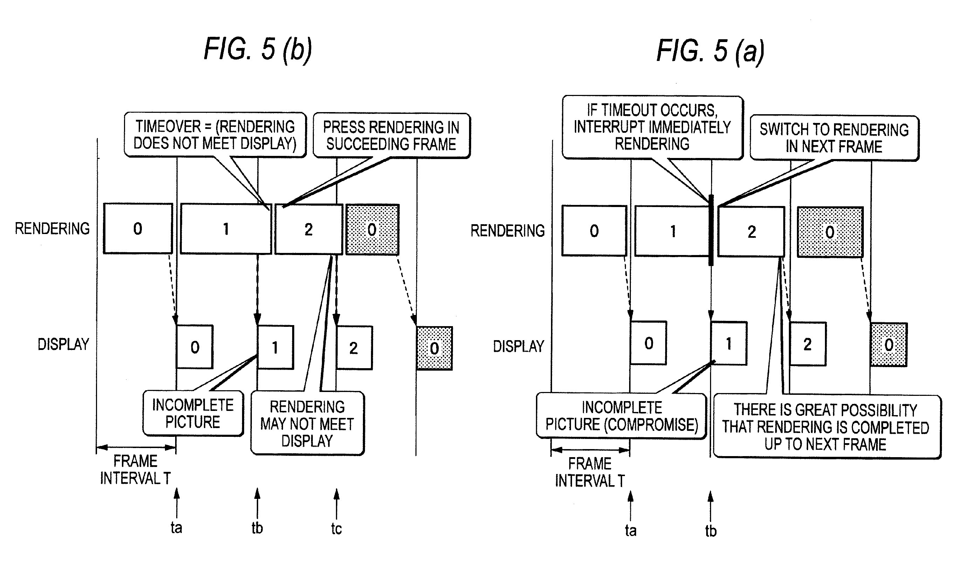

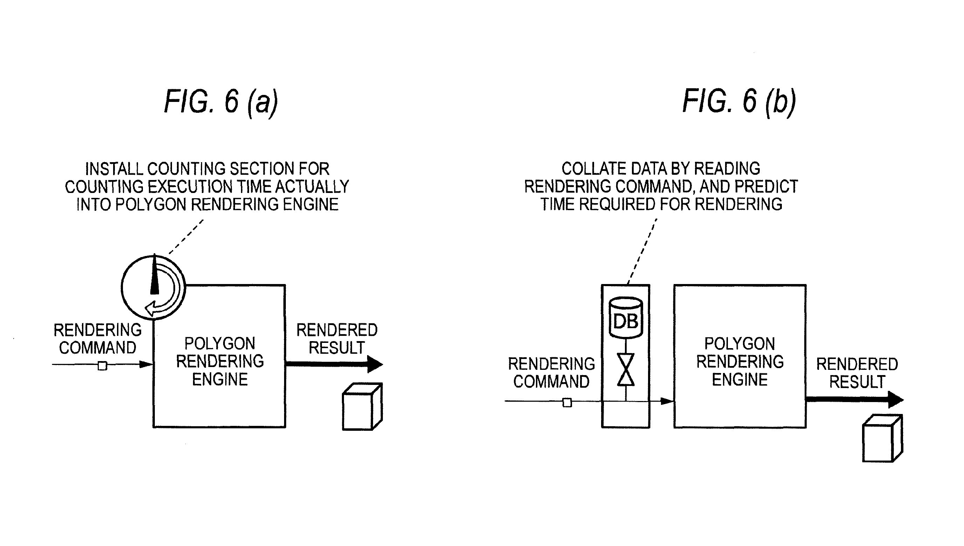

FIG.1 is a block diagram showing an example of a hardware configuration of a graphics display device, into which a graphics rendering device in the embodiment is installed, in an embodiment. FIG.2 is flowcharts showing examples of operations of a principal portion of the device shown in FIG.1. FIG.3 is a time chart showing an example of an operation of the device shown in FIG.1 and an operation in the prior art. FIG.4 is a time chart showing an example of an operation of the device shown in FIG.1. FIG.5 is a time chart showing an example of an operation of the device shown in FIG.1 and an operation in the prior art. FIG.6 is a block diagram showing a concrete example of constituent elements used to detect a rendering necessary time.

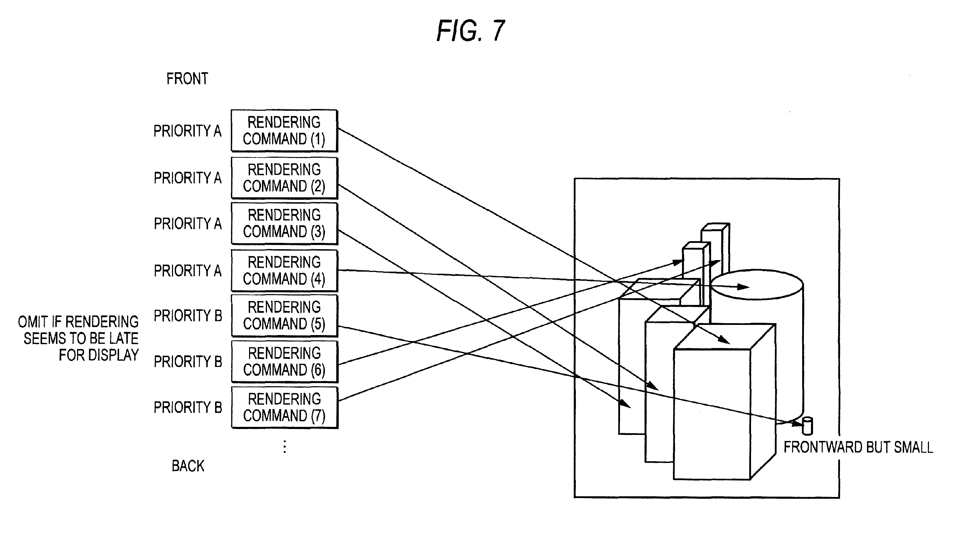

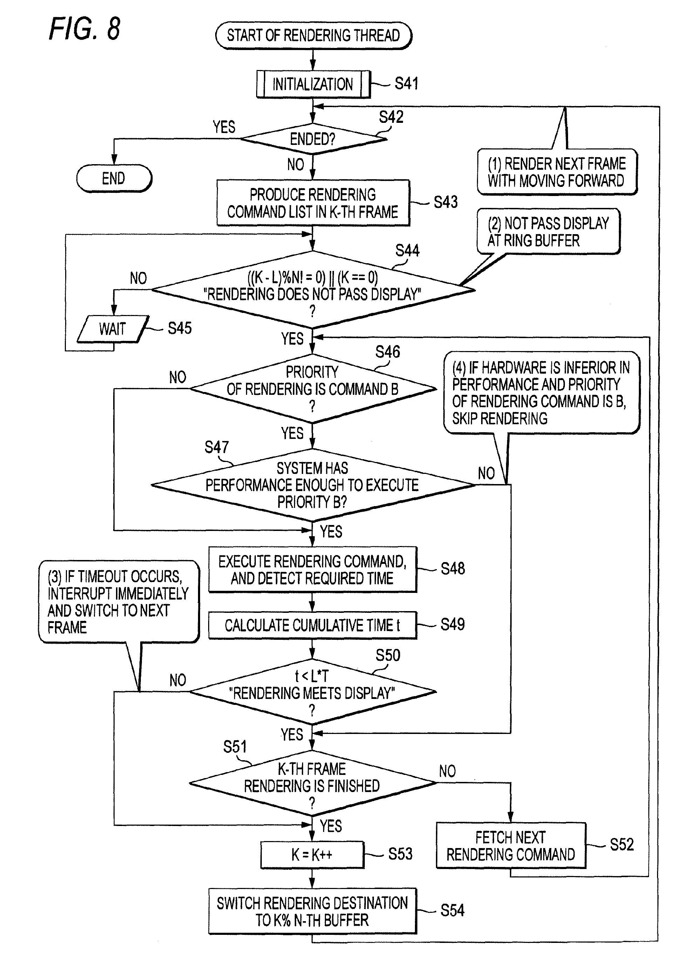

FIG.7 is a schematic view showing a configurative view of a rendering command list. FIG.8 is a flowchart showing an example of an operation of the principal portion of the device shown in FIG.1.

The graphics display device shown in FIG.1 includes a graphics processing portion 10, a rendering command holding portion 20, a display device 30, and a ring buffer 40. Also, the graphics processing portion 10 includes a microprocessor (CPU) 11, a polygon rendering engine 12, a rendering time detecting portion 13, a rendering-time accumulation calculating portion 14, a rendering counter 15, a displaying engine 16, and a display counter 17.
In this case, the graphics display device corresponds to a cellular phone, PDA, a hand-held game machine, or the like, for example.

The graphics processing portion 10 is a hardware that executes various processes concerning the graphics. The display device 30 is a display device equipped with a two-dimensional display screen like a liquid crystal display device, for example. The rendering command holding portion 20 is a memory device for holding data of a rendering command list 21 in which rendering commands corresponding to respective display elements (graphics such as polygons, or the like) to be displayed in each frame of a screen are aligned as a table. The ring buffer 40 is a memory device for holding data of display contents, which are to be displayed on the screen of the display device 30, every frame.

In the concrete example shown in FIG.1, three memory areas 41(0), 41(1), 41(2) are provided on the ring buffer 40. The memory areas 41(0), 41(1), 41(2) can hold the data corresponding to the display contents of independent frames respectively. Three memory areas 41 (0), 41(1), 41 (2) are used sequentially and cyclically in a situation that these areas are correlated with respective display frames, which appear continuously, of the display device 30, and as a result function as the ring buffer. That is, these memory areas 41 (0), 41(1), 41 (2) are correlated with respective display frames in sequence of 41(0), 41(1), 41(2), 41(0), 41(1), 41(2),···.

The microprocessor 11 produces rendering commands as the data that decide the display contents, and stores them in the rendering command holding portion 20 as the rendering command list 21. Also, the microprocessor 11 executes the overall control of the graphics processing portion 10.

The polygon rendering engine 12 is a hardware used exclusively for the rendering process, and executes the polygon rendering in accordance with respective rendering commands existing in the rendering command list 21. The result rendered by the polygon rendering engine 12 is stored in any one of the memory areas 41 on the ring buffer 40. Also, when the polygon rendering engine 12 fetches the rendering command from the rendering command list 21, the data of the rendering command are transferred from the rendering command holding portion 20 to the polygon rendering engine 12 by means of DMA (Direct Memory Access) without intervention of the microprocessor 11. Also, the polygon rendering engine 12 is designed to originate an interrupt to the process of the microprocessor 11 when a series of rendering processes are completed in the polygon rendering engine 12.

The displaying engine 16 is a dedicated hardware that executes the process to display the rendered contents on the screen of the display device 30. Concretely, the displaying engine 16 carries out the process to transfer the contents (data) in respective memory areas 41 on the ring buffer 40 to the memory on the display device 30 at a high speed. Also, the displaying engine 16 gives a predetermined completion informing interrupt to the microprocessor 11 as a frame synchronization interrupt every time when the process of transferring the data of one frame to the display device 30 is completed.

The display counter 17 is a counter that counts the number of frames that have already been displayed on the display device 30. Here, the contents of the display counter 17 are indicated with a variable L. The rendering counter 15 is a counter that counts the number of frames in which the rendering process has been ended. Here, the contents of the rendering counter 15 are indicated with a variable K. The rendering time detecting portion 13 counts a rendering necessary time required when the polygon rendering engine 12 executes each rendering command. The rendering-time accumulation calculating portion 14 executes the accumulated processes concerning the rendering necessary time that the rendering time detecting portion 13 counts.

A concrete example of controlling procedures concerning major processes of the graphics display device shown in FIG.1 is shown in FIG.2. The processes shown in FIG.2 are controlled by the microprocessor 11, and are constructed by a rendering thread expressing the process contents of programs being operated mutually independently and a display thread.

First, an algorithm of the display thread shown in FIG.2 will be explained hereunder. In this case, the displaying operation cannot be started unless the displayable rendering data are completed. Therefore, it is common that either the start of the display thread is delayed from the rendering thread by a specified time or the display thread is started after the displayable data are completed.

When the display thread is started, the contents (L) of the display counter 17 are initialized to 0 in an initializing step (S31). Then, the process goes through an end deciding step (S32), and it is waited that an interrupt of the display frame generated in a period T enters into the microprocessor 11 (step S33). In this case, the period T represents an update period of the display frame, and is set to a predetermined time of about 1/60 second, for example.

When the interrupt of the display frame enters, the process goes to step S34. Then, the data being held in the ring buffer 40 (any one of the memory areas 41(0), 41(1), 41(2)) that corresponds to an index (variable) obtained when the contents (L) of the display counter 17 are indicated by a residue system using the cyclic number N (in an example in FIG.1, N=3) of the ring buffer 40 as a base are transferred to the display device 30 by using the displaying engine 16. Then, the contents of the data are displayed on the screen.

The contents (L) of the display counter 17 are incremented in step S35. Then, an index of the ring buffer 40 that is to be displayed next is updated (S36). This index is obtained when the value of the contents (L) of the display counter 17 are indicated by the residue system using the cyclic number N as a base, as described above. Then, the process goes back to step of deciding the end of display (S32). When the end of display is detected, the display thread is ended.

In brief, when the display thread shown in FIG.2 is executed repeatedly, the contents of the memory areas 41(0), 41(1), 41(2), 41(0), 41(1), 41(2),··· of the ring buffer 40 are transferred sequentially to the display device 30 and then displayed on the screen every time when the interrupt of the display frame enters. Such a case is explained that the contents of the memory areas 41(0), 41(1), 41(2), 41(0), 41(1), 41(2),··· of the ring buffer 40 are transferred sequentially every time when the interrupt of the display frame enters. In this case, such a configuration may be employed that the contents of the memory areas 41(0), 41(1), 41(2), 41(0), 41(1), 41(2),··· of the ring buffer 40 may be transferred sequentially when the interrupt of the display frame enters the predetermined number of times. As the interruption period of the display frame, 1/60 second used as the scanning period of the liquid crystal screen is popular, and thus the image is updated 60 times for one second. In this case, the image may be updated 30 times, 20 times, or 15 times for one second, depending on the contents of the display image. In order to adjust the number of times the image is updated for one second in this manner, if such a configuration may be employed that the contents of the memory areas 41 of the ring buffer 40 are transferred sequentially when the interrupt of the display frame enters two times, the image can be updated 30 times for one second. Also, if such a configuration may be employed that the contents of the memory areas 41 of the ring buffer 40 are transferred sequentially when the interrupt of the display frame enters three times, the image can be updated 20 times for one second. Also, if such a configuration may be employed that the contents of the memory areas 41 of the ring buffer 40 are transferred sequentially when the interrupt of the display frame enters four times, the image can be updated 15 times for one second. At this time, the display counter L may be updated when the interrupt of the display frame enters two times, three times, or four times.

Next, an algorithm of the rendering thread shown in FIG.2 will be explained hereunder. Here, in respective steps in the comparing process shown in FIG.2, "!=" denotes a comparison that is made to decide whether or not values in a left side and a right side are unequal to each other, "∥" denotes a logical sum (OR), and "==" denotes a comparison that is made to decide whether or not values in a left side and a right side are equal to each other. (K=0)

When the rendering operation is started, various counters, and others are cleared to 0 in initializing step (S11). Then, the process goes through end deciding step (S12), and then the microprocessor 11 produces the rendering command list 21 in the K-th frame (S13). Then, the rendering command list 21 is saved in the rendering command holding portion 20 shown in FIG.1.

Then, in step S14, an index indicating the destination memory area 41 of the ring buffer 40 that the polygon rendering engine 12 renders at present (variable indicating the writing position, i.e., the rendering position) is compared with an index indicating the memory area 41 from which the display is made or from which the display data are now transferred (variable indicating the reading position, i.e., the display position), and then it is decided whether or not the rendering index does not pass the display index on a mechanism of the ring buffer 40.

As a simple deciding method, when a difference value of the index between the rendering and the display is not 0 when viewed based on the residue system using the cyclic number N as a base (if the condition of ((K-L)%N!=0) is satisfied), no overlapping is caused on the ring buffer 40. Therefore, neither the passing nor the catching-up is caused, and thus the rendering operation is carried out safely. The rendering process is applied to the empty area of the ring buffer 40 (area in which the unnecessary data that have already been displayed are held). In this case, the process goes through unconditionally this deciding step (S14) and goes to next step to execute the rendering command immediately after the rendering thread is started, i.e., when the contents (K) of the rendering counter 15 are 0. When the catching-up is detected as the result of decision, the process does not go to step of executing the rendering command and enters into waiting step (S15) until the display counter L is updated and the overlapping of the operation between the rendering buffer and the display buffer is eliminated. The reason why the waiting is needed will be described later.

Next, step of executing the rendering command will be explained hereunder. When the polygon rendering engine (rendering engine) 12 receives the command, which allows the execution of the rendering operation, from the microprocessor 11, this engine acquires a command sequence of the rendering command list 21 from the buffer of the rendering command holding portion 20 via the DMA transfer, and executes the rendering process in accordance with the command contents (S16).

Also, as shown in FIG.1, the rendering time detecting portion 13 is connected to the polygon rendering engine 12, and the rendering time detecting portion 13 monitors an operation state of the polygon rendering engine 12 and a time. Then, the rendering time detecting portion 13 detects a required time δ until the command is ended after the start of execution every rendering command, in step S16.

Also, the rendering-time accumulation calculating portion 14 connected to the rendering time detecting portion 13 accumulates sequentially a required time δ that the rendering time detecting portion 13 detects at respective points of time, and calculates a cumulative time t (S17). This cumulative time t denotes a cumulative elapsed time from a time point when the rendering thread in FIG.2 is started.

Then, the process goes to next step in which it is decided whether or not the process in the K-th frame that the polygon rendering engine 12 is rendering now can be completed up to the display timing. That is, in step S18, the cumulative time t that the rendering-time accumulation calculating portion 14 calculated is compared with (L×T) that indicates a time at which the display is made in the display thread. Where L is a value of the display counter, and T is a frame period of the display.

If the time t is smaller than (L×T), the rendering can be ended before the time at which the display is made, and thus the process goes to next step S19. If it is decided that the rendering cannot be ended before the display time, it is decided that the continuation of rendering is meaningless because the data being rendered now with much effort are not completed up to the display time. The rendering of the frame that is processed at present is interrupted, and the process jumps to an updating step of the counter K (S21) to go to the rendering operation in the next frame. Detailed explanation of the reason why the jump is executed will be given later with reference to FIG.5.

If t is smaller than (L×T), it is decided whether or not the K-th frame that is rendered at present is completed, i.e., whether or not the execution of all rendering commands on the rendering command list 21 is ended, in next step S19. If it is decided that the K-th frame is not completed, the process goes to step S20 to execute the next rendering command. Then, the next rendering command is fetched from the rendering command list 21. Then, the processes subsequent to step S16 are repeated like a loop.

If it is decided that the rendering in the K-th frame is completed, the process goes to step S21 from step S19, and then the value K of the rendering counter 15 is updated. Then, the target buffer (one concerned memory area 41 on the ring buffer 40) in which the rendering is executed in next step S22 is switched to the buffer corresponding to the next index. In this case, the value of this index can be obtained by indicating the value K of the rendering counter 15 by using the residue system using N as a base (K%N).

In the process of the rendering thread shown in FIG.2, the target buffer as the rendering destination is switched, then the process is looped to an end deciding step (S12) to proceed the rendering operation in the next frame without break, and then the rendering in the next frame is continued unless the process arrives at the final frame. The reason for this will be explained with reference to FIG.3 hereunder.

FIG.3 shows a concrete example of timings of the render operation and the display operation in the graphics display device shown in FIG.1. FIG.3(a) shows a timing when the operation shown in FIG.2 is executed, and FIG.3(b) shows a timing when the normal (conventional) operation is executed.

In an operation example shown in FIG.3(b), the rendering process is started at a start time of the frame every frame, and the rendering process is ended before a time that is allowed for one frame (within a frame interval T) has elapsed. Then, the data of the display contents whose rendering process is ended are displayed to coincide with the timing at which the next frame is started. Therefore, the data of the rendering object (corresponding to the contents in the rendering command list 21) must be prepared previously a little more small such that the rendering process can be ended without fail before an elapsed time exceeds the frame interval T. As a result, a "remaining time" during which the polygon rendering engine 12 stops the rendering process occurs until the next frame is started after the rendering process in each frame is completed. Accordingly, a full span of the frame interval T cannot be utilized effectively in executing the rendering process, and a large amount of rendering commands cannot be executed even though the polygon rendering engine 12 whose processing capabilities are high is employed. As a result, the execution of the effective rendering process can be realized by executing the operation shown in FIG.2.

Namely, in the operation example shown in FIG.3(a), after the polygon rendering engine 12 ended the rendering process in one frame, such polygon rendering engine 12 starts the rendering process in the subsequent frame not to wait the timing at which the next frame is started. As a consequence, the "remaining time" shown in FIG.3(b) does not occur, and a full span of the limited time (frame interval T) can be utilized effectively to execute the rendering process. In the graphics display device shown in FIG.1, a plurality of memory areas 41 are provided to the ring buffer 40, and thus the rendering process regarding a plurality of frames except the frame that is displayed at present can be executed simultaneously. Therefore, when a large amount of rendering commands are prepared in advance as the rendering command list 21, complicated and sophisticated pictures can be rendered by using effectively the "remaining time".

However, it is possible that, because the number of memory areas 41 on the ring buffer 40 is finite, the problem shown in FIG.4(b) is caused unless the special control is applied. That is, in an example shown in FIG.4(b), the case where the polygon rendering engine 12 can execute the display contents in three (N) frames or more in one frame time (frame interval T) is assumed. Therefore, after the display contents are written sequentially in the memory areas 41(0), 41(1), 41(2) within a render time of one frame, the display contents in the succeeding frame must be written into the memory area 41 (0) before the contents of the memory area 41 (0) are displayed on the screen. As a result, there is a possibility that the display contents written previously into the memory area 41 (0) are destroyed by the overwriting.

Therefore, an outbreak of such problem can be prevented by executing the processes in the rendering thread shown in FIG.2. That is, when it is determined in step S14 in FIG.2 whether or not the rendering process does not pass the display, i.e., whether or not an empty area except the area in which the data whose display is not completed are not written exists in the ring buffer 40, the destruction of the data caused by the above overwriting can be prevented.

For example, in an operation example shown in FIG.4(a), after the polygon rendering engine 12 writes the display contents sequentially in the memory areas 41(0), 41(1), 41(2) within a render time of one frame, such polygon rendering engine 12 interrupts the rendering process and stands by until the timing at which the next frame is started, to prevent the destruction of the data in the memory area 41 (0)(corresponding to S15 in FIG.2).

Meanwhile, even in the situation that the tolerance is established to some extent to the rendering time in one frame, such a case happens that the completion of the rendering process cannot catch the display time of that frame when the picture (contents to be displayed) becomes more complicated and sophisticated.

For example, an operation example shown in FIG.5(b), the rendering of the frame that is to be processed in the interval (T) between a time ta and a time tb is not completed at a time tb. The rendering process cannot be ended up to the display start timing (tb), and thus an incomplete picture is displayed on the screen. Further, since the rendering process in the preceding frame is still continued at a time tb, the start of the rendering process in the frame that is to be processed in the interval (T) between a time tb and a time tc is delayed. Therefore, it is highly possible that the rendering process in the succeeding frame cannot catch the display timing. As a result, the breakdown of the contents of the picture being displayed actually is caused.

However, occurrence of such problem can be avoided by executing step S18 in the process of the rendering thread shown in FIG.2. In other words, either when an execution time of the rendering process in some frame goes past the timing at which the concerned frame is displayed or when it is decided that the rendering process is not completed until that timing, the execution of the subsequent rendering command is omitted and then the process goes to the rendering process in the succeeding frame.

For example, in an operation example shown in FIG.5(a), the rendering operation in the frame that is to be processed in the interval (T) between a time ta and a time tb is not ended up to a time tb. Therefore, the rendering in that frame is interrupted at a time point when an elapsed time goes past a time tb, and then the process goes to the rendering operation in the succeeding frame. As a result, the incomplete picture in the particular frame that does not fit into the interval between a time ta and a time tb is displayed. In this event, such incomplete picture has no influence upon the rendering operation in the succeeding frame, and the breakdown of the display contents is suppressed to the lowest minimum.

In the step S18 of the rendering thread shown in FIG.2, the rendering process is interrupted at a time point when this rendering process goes past the timing at which the display is started. In this case, if the compared contents in the step S18 is changed, the rendering process may be interrupted before this rendering process goes past the timing at which the display is started.

A configurative example of the rendering time detecting portion 13 that can be installed into the graphics display device shown in FIG.1 is shown in FIG.6. Like the configuration shown in FIG.1, a configurative example shown in FIG.6(a) is constructed such that the rendering time detecting portion 13 monitors an operation state of the polygon rendering engine 12 and a time, and measures a required time required for the actual rendering process.

In contrast, in a configurative example shown in FIG.6(b), such a case is assumed that the rendering time detecting portion 13 grasps the contents of respective rendering commands that are input from the rendering command list 21 to the polygon rendering engine 12, and estimates a required time necessary for the rendering process from the contents. Concretely, a database for holding in advance the constants, which represent a time required for executing that command respectively, every type of the rendering command is provided, and the rendering time detecting portion 13 accesses the database based on the type of the detected rendering command and estimates a required time for the rendering operation. Here, an actual rendering required time changes depending on the performance of the employed polygon rendering engine 12. However, even when a rendering required time is detected from plural types of polygon rendering engines 12 whose performance is different respectively, the constants on the database may be corrected by using a correction factor that responds to the performance of the actually employed polygon rendering engine 12, and as a result an exact required time can be estimated.

By the way, when the (large amount of) rendering commands that are too heavy in contrast to the performance of the employed polygon rendering engine 12 should be processed, all rendering commands cannot be processed within a limited time that is allowed for each frame. Therefore, such a case occurs, as shown in FIG.5(a), for example, that the polygon rendering engine 12 cannot but interrupt the rendering operation in the middle. In such case, the incomplete picture is displayed depending on the performance of the polygon rendering engine 12 regardless of the will of the contents creator. As a result, it is impossible to avoid such a situation that a looking layout of the display contents becomes worse.

A method of solving such problem will be explained hereunder. As shown in FIG.7, for example, the rendering command list 21 held on the rendering command holding portion 20 contains priority information, which are correlated with the rendering command respectively, together with the rendering commands. In an example shown in FIG.7, the case where two types of priorities consisting of a "priority A" and a "priority B" can be assigned is assumed. For example, the "priority A" as a high priority is assigned to the rendering command that is used to render an relatively large element, a conspicuous element like an element located on the front side of other elements, or an element having a great influence, among the display elements displayed on the screen. On the contrary, the "priority B" as a low priority is assigned to the rendering command that is used to render an relatively small element, or an inconspicuous element like an element located on the back side of other elements.

As a consequence, when the application program such as the game contents, or the like are executed in the device such as the cellular phone, or the like having many restrictions on the hardware performance, the contents creator can utilize the assignment of the priority to implement the way of decimation of the rendering operation and the keeping of the display rate after such creator took a looking layout into consideration.

That is, the rendering commands 1 to 4 with the "priority A" are handled preferentially. However, such a control can be applied that, when the rendering operation is not quick to keep the frame rate in light of the essential potential of the polygon rendering engine 12, the rendering command 5 et seq. with the "priority B" may skip over the rendering operation and may be shifted to execute the next rendering command. In other words, the rendering commands to which the "priority B" is assigned may be specified from the beginning by the contents creator as "the object that can notify the player of the screen intention unless such object is particularly provided, or the object that can give a rich expression if such object is provided but does not cause any trouble unless such object is provided" in constituting one screen. Accordingly, the display contents that reflect the will of the content creator can be decided.

When the rendering command list 21 containing the information of the priority as shown in FIG.7 is present, the control contents of the microprocessor 11 may be changed like an operation example shown in FIG.8 instead of the operation example shown in FIG.2. The operation example shown in FIG.8 is different from FIG.2 in that step S46 and step S47 are added, but remaining steps are similar to those in FIG.2.

In step S46 in FIG.8, it is decided to which one of the "priority A" and the "priority B" the priority of the rendering command of the process object corresponds. If the priority is the "priority B", the process goes to a next step S47. If the priority is the "priority A", the process goes to next step S48.

In step S47, it is decided whether or not the hardware of this device that includes the polygon rendering engine 12 as a main body is equipped with the performance that is enough to execute the rendering command with the "priority B". In other words, when the hardware whose capabilities are high is installed, the hardware has a margin in process. Thus, the high-quality picture containing a large quantity of information can be displayed by executing the rendering commands having the relatively low priority. In contrast, when the hardware whose capabilities are low is installed, the hardware does not have a margin in process and thus the execution of the rendering command with the low priority may be omitted. As a result, such a control can be applied that an occurrence of the situation in which the process is not finished in time can be prevented and also the lowest minimum rendering commands with the high priority can be executed without omission to reflect the will of the contents creator.

In this case, the performance of the hardware that is detected in step S47 can be specified based on the type of the employed polygon rendering engine 12 and its operation frequency. Therefore, for example, the information indicating the type of the polygon rendering engine 12 and the information indicating the operation frequency of the polygon rendering engine 12 may be registered in advance in ROM (Read Only Memory) as the basic information. Then, upon executing the step S47, the performance of the hardware may be detected based on the information read from the ROM, and then the result may be compared with the constants (threshold values) that are correlated in advance with the "priority B".

In the concrete examples shown in FIG.7 and FIG.8, the case where two types of the "priority A" and the "priority B" are prepared as the priority is assumed. But three types or more of the priorities may be assigned to respective rendering commands. In such case, the processes similar to those in steps S46, S47 in FIG.8 may be added further in response to the assignable number of priorities.

The present invention is explained in detail with reference to the particular embodiment. But it is apparent for those skilled in the art that various variations and modifications can be applied without departing from a spirit and a scope of the present invention.

### Industrial Applicability

The present invention can make the drawing engine exhibit the processing abilities to maximum even when the high-quality graphics should be displayed by executing the 3D game application program, or the like in the equipment, for example, the cellular phone terminal or the mobile information terminal (PDA), into which the high-performance polygon rendering engine is not always installed. Therefore, even when a time that is allowable for the rendering operation in each frame is short, the display frame rate can be kept and also the breakdown of display contents can be suppressed.

## Claims

1. A graphics rendering device for sequentially rendering a plurality of display frames that appear sequentially in time series, comprising:
a frame buffer that has a plurality of areas for plural frames, each of the areas storing data of display contents displayed in one display frame;
a rendering frame counter that counts a value which is changed in response to a number of frames in which the rendering is ended;
a display frame counter that counts a value which is changed in response to a number of frames in which a display on a screen is completed within respective frames whose rendering is ended;
a rendering necessary-time predicting portion that predicts a rendering required time; and
a rendering omission controlling portion that determines whether or not the rendering is completed before the display, based on the value of the display frame counter, a display period of the frame, and a rendering required time predicted by the rendering necessary-time predicting portion, and performs a rendering process of the next frame by omitting an execution of a succeeding rendering command when it is determined that the rendering is not completed before the display.

2. The graphics rendering device according to claim 1, further comprising:
a rendering controlling portion that determines whether or not an empty area is present on the frame buffer based on the value of the rendering frame counter and the value of the display frame counter, and permits the rendering of the display element in the next frame when it is determined that the empty area is present,
wherein when the rendering controlling portion permits the rendering, the rendering necessary-time predicting portion predicts a rendering required time.

3. The graphics rendering device according to claim 1, wherein the rendering necessary-time predicting portion estimates a required time for the rendering in one frame based on a list of the rendering commands indicating the rendering contents in the concerned frame, as a cumulative value of the required times detected when respective rendering commands contained in the list are executed.

4. The graphics rendering device according to claim 1, wherein the rendering necessary-time predicting portion estimates a required time for the rendering in one frame based on the list of the rendering commands indicating the rendering contents in the concerned frame, as a cumulative value of constants indicating execution required times that are assigned in advance to respective rendering commands contained in the list.

5. The graphics rendering device according to claim 1, further comprising:
a drawing command list holding portion that holds the list of the rendering commands indicating the rendering contents in respective frames, and holds priority information indicating categories concerning two types or more of priorities in the list every rendering command,
wherein the rendering controlling portion determines whether or not respective rendering commands contained in the list held in the drawing command list holding portion are able to execute the rendering, based on the correlated priority and processing capabilities of a rendering engine which executes the rendering, and omits an execution of the rendering commands which satisfy predetermined conditions.

6. A graphics rendering method of sequentially rendering a plurality of display frames which appear sequentially in time series, comprising:
utilizing a frame buffer having a plurality of areas for plural frames, each of the areas storing data of display contents displayed in one display frame;
counting a first value which is changed in response to a number of frames in which the rendering is ended;
counting a second value which is changed in response to a number of frames in which a display on a screen is completed within respective frames whose rendering is ended; and
determining whether or not the rendering is completed before the display, based on the second value, a display period of the frame, and a predicted rendering required time, and performing a rendering process of a next frame by omitting an execution of a succeeding rendering command when it is determined that the rendering is not completed before the display.

7. A graphics display device into which the graphics rendering device set forth in claim 1 is installed.
